(19) Europäisches Patentamt
European Patent Office
Office européen des brevets

(11) EP 0 736 606 B1

(12) # FASCICULE DE BREVET EUROPEEN

(45) Date de publication et mention de la délivrance du brevet:
**02.01.2002 Bulletin 2002/01**

(51) Int Cl.$^7$: **C12P 41/00**, C12P 7/62, C07C 31/20, C07C 59/01

(21) Numéro de dépôt: **96400700.9**

(22) Date de dépôt: **01.04.1996**

(54) **Procédé de préparation enzymatique d'un intermédiaire de synthèse de la béfloxatone**

Verfahren zur enzymatischen Herstellung eines Synthese-Zwischenprodukt von Befloxatone

Process for the enzymatic preparation of intermediates for the synthesis of befloxatone

(84) Etats contractants désignés:
**AT BE CH DE DK ES FI FR GB GR IE IT LI LU NL PT SE**

(30) Priorité: **07.04.1995 FR 9504141**

(43) Date de publication de la demande:
**09.10.1996 Bulletin 1996/41**

(73) Titulaire: **SANOFI-SYNTHELABO**
**75013 Paris (FR)**

(72) Inventeurs:
- **Tixidre, Arlette**
  **91400 Orsay (FR)**
- **Zard, Lydia**
  **91190 Gif sur Yvette (FR)**
- **Rossey, Guy**
  **78960 Voisins le Bretonneux (FR)**
- **Bourbon, André**
  **28260 La Chaussee d'Ivry (FR)**

(74) Mandataire: **Thouret-Lemaitre, Elisabeth et al**
**Sanofi-Synthélabo Service Brevets 174, avenue de France**
**75013 Paris (FR)**

(56) Documents cités:

| | |
|---|---|
| **EP-A- 0 334 966** | **EP-A- 0 424 244** |
| **EP-A- 0 577 446** | **EP-A- 0 709 465** |
| **EP-A- 0 747 487** | **EP-A- 0 774 452** |
| **WO-A-89/02916** | **WO-A-95/34525** |
| **WO-A-96/12035** | |

- **J. ORG. CHEM. (1987), 52(15), 3211-17 CODEN: JOCEAH;ISSN: 0022-3263, 1987, XP002008200 LIN, JENQ TAIN ET AL: "A microbially based approach for the preparation of chiral molecules possessing the trifluoromethyl group"**
- **CHEMICAL ABSTRACTS, vol. 116, no. 12, 30 Mars 1992 Columbus, Ohio, US; abstract no. 127023d, page 718; XP002008202 & JP-A-03 254 694 (KURITA WATER IND.) 13 Novembre 1991**
- **CHEMICAL ABSTRACTS, vol. 113, no. 7, 13 Août 1990 Columbus, Ohio, US; abstract no. 57476r, page 560; XP002008203 & JP-A-02 040 343 (MEITO SANGYO) 9 Février 1990**
- **CHEMICAL ABSTRACTS, vol. 120, no. 3, 17 Janvier 1994 Columbus, Ohio, US; abstract no. 29497, MYAZAWA, TOSHIFUMI ET AL: "Microbial manufacture of optically active halogen-containing alcohols" XP002008204 & JP-A-05 219 986 (SHOWA SHELL SEKIYU, JAPAN)**
- **CHEMICAL ABSTRACTS, vol. 110, no. 13, 27 Mars 1989 Columbus, Ohio, US; abstract no. 113136g, KITAZUME ,TOMOYA: "A microbially based approach for the preparation of fluorinated ferroelectric liquid crystals" page 552; XP002008205 & BIO IND, vol. 5, no. 10, 1988, JAPAN, pages 733-740,**
- **HELVETICA CHIMICA ACTA., vol. 72, no. 4, 1989, BASEL CH, pages 793-799, XP002008201 EHRLER,JUERG ET AL.: "Notiz über mikrobiologische Umsetzungen mit Halobacterium halobium: Reduktion von 3-Oxobutansäure-ethylester und Hydrolyse von 3-Hydroxybutansäure -ethylester.Cooperative Effekte von Reduktase und Hydrolase."**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 736 606 B1

## Description

La présente invention a pour objet un nouveau procédé de préparation du 4,4,4-trifluoro-3(R)-hydroxybutanoate d'éthyle et son utilisation pour la préparation du 4,4,4-trifluoro-1,3(R)-butanediol, intermédiaire de synthèse de la 3-[4-(4,4,4-trifluoro-3(R)-hydroxybutoxy)phényl]-5(R)-méthoxyméthyl-2-oxazolidinone (béfloxatone).

La béfloxatone est un composé utile comme inhibiteur de la monoamine oxydase A, elle est décrite dans la demande de brevet EP 90 402892.5 (Pub. EP-A-0 424 244).

Selon le procédé décrit dans cette demande de brevet, la béfloxatone est obtenue par réaction de la 3-(4-hydroxyphényl)-5(R)-méthoxyméthyl-2-oxazolidinone avec le 4,4,4-trifluoro-1-tosyloxy-3(R)-butanol, ce dernier composé étant lui-même préparé à partir du (±)-4,4,4-trifluoro-3-hydroxybutanoate d'éthyle, par l'intermédiaire de l'acide (±)-4,4,4-trifluoro-3-hydroxybutanoïque, de l'acide 4,4,4-trifluoro-3(R)-hydroxybutanoïque et du 4,4,4-trifluoro-1,3 (R)-butanediol.

Selon ce procédé, on transforme le (±)-4,4,4-trifluoro-3-hydroxybutanoate d'éthyle en sel de sodium de l'acide butanoïque correspondant qui est hydrolysé pour donner l'acide (±)-4,4,4-trifluoro-3-hydroxybutanoïque, on fait réagir cet acide avec la (S)-α-méthylbenzylamine, on sépare par cristallisation dans l'éthanol, le sel de (S)-α-méthyl benzylamine de l'acide 4,4,4-trifluoro-3(R)-hydroxybutanoïque à partir du mélange de diastéréoisomères formés, puis on hydrolyse ce sel en milieu acide pour obtenir l'acide 4,4,4-trifluoro-3(R)-hydroxybutanoïque.

Le 4,4,4-trifluoro-1,3(R)-butanediol est ensuite obtenu par réduction de l'acide 4,4,4-trifluoro-3(R)-hydroxybutanoïque.

La présente invention concerne la préparation du 4,4,4-trifluoro-3(R)-hydroxybutanoate d'éthyle, précurseur du 4,4,4-trifluoro-1,3(R)-butanediol, utilisé comme intermédiaire de synthèse de la béfloxatone.

De *J. Org. Chem. (1987), 52(15), 3211-17* est connu un procédé de préparation enzymatique selon le schéma suivant :

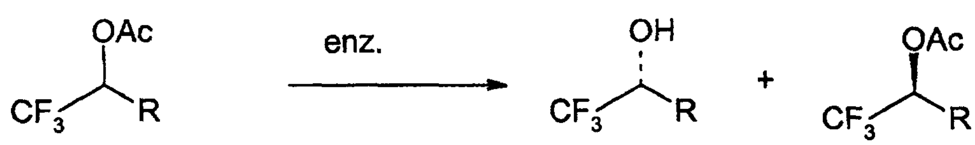


et en particulier, d'après le tableau I, l'obtention de

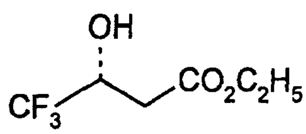

est connue, lorsque l'enzyme est une lipase-MY.

Ce procédé enzymatique diffère en particulier du procédé selon la présente invention par le produit de départ. Ainsi dans *J. Org. Chem. (1987), 52(15), 3211-17,* la fonction ester à hydrolyser se trouve en α du centre asymétrique à dédoubler tandis que le produit de départ de la présente invention présente une fonction ester en β de ce centre à dédoubler.

De *Chemical Abstracts, vol. 116, no. 12, 30 Mars 1992 ; abstract no. 127023d, page 718 ; & JP-A-03 254 694* est connu en particulier, un procédé de préparation enzymatique selon le schéma suivant :

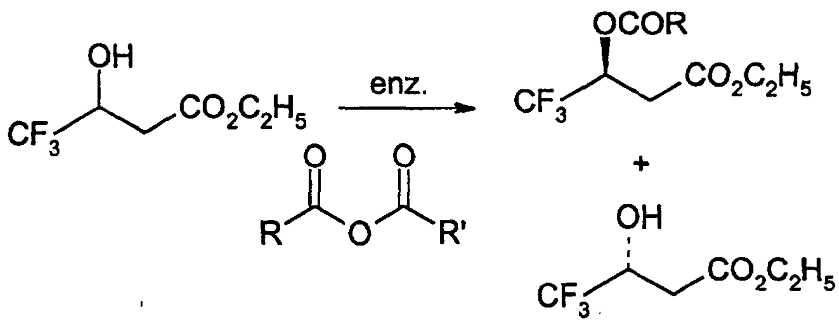

lorsque l'enzyme est une lipase issue de la souche Pseudomonas kWI-S6. Ce procédé enzymatique est une transesterification et non une hydrolyse, le procédé de la présente invention diffère donc du procédé enzymatique décrit dans *Chemical Abstracts, vol. 116, no. 12, 30 Mars 1992 ; abstract no. 127023d, page 718 ; & JP-A-03 254 694.*

De *Chemical Abstracts, vol. 113, no. 7, 13 Août 1990 ; abstract no. 57476r, page 560 ; JP-A-02 040 343* est connu un procédé de préparation enzymatique de polymères consistant en des polyesters fluorés. Il n'y est donc nullement question de l'obtention du 4,4,4-trifluoro-3(R)-hydroxybutanoate d'éthyle. Le procédé de la présente invention diffère donc du procédé enzymatique décrit dans *Chemical Abstracts, vol. 113, no. 7, 13 Août 1990 ; abstract no. 57476r, page 560 ; JP-A-02* 040 343.

De *EP-A-0577 446* est connu un procédé de préparation enzymatique selon le schéma suivant (page 2, lignes 25 à page 4 ligne 31)

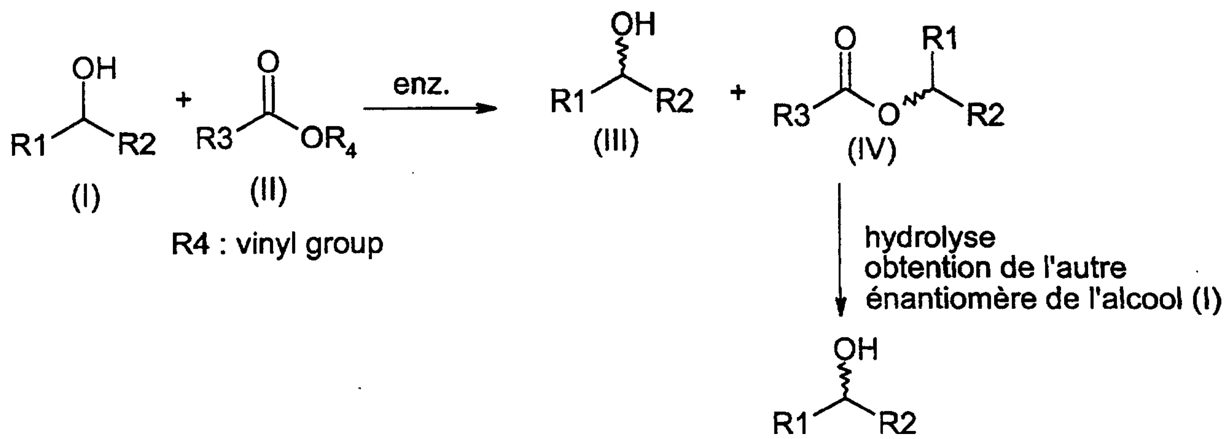

(I) (II) (III) (IV)
R4 : vinyl group

où 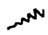 signifie que le composé comporte un centre chirale (R) ou (S) et où le type d'énantiomère obtenu dépend de l'enzyme employée.

Ce procédé nécessite donc l'utilisation d'un agent acylant (II), ainsi il ne s'agit pas d'une hydrolyse de la fonction ester telle que dans le procédé selon la présente invention.

De *EP-A-0 334* 966 est connu un procédé enzymatique de préparation selon le schéma suivant, décrit à la page 7, lignes 1 à 15 et l'exemple (5), page 6, lignes 27 à 41 de *EP-A-0 334 966:*

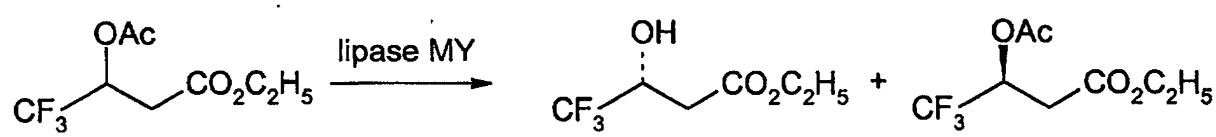


Ce procédé enzymatique diffère donc de celui de la présente invention dans la structure de produits de départ.

Selon l'invention, le 4,4,4-trifluoro-3(R)-hydroxybutanoate d'éthyle est obtenu par hydrolyse enzymatique sélective de la fonction ester du (±) -4,4,4-trifluoro-3-hydroxybutanoate d'éthyle au moyen d'une lipase, qui conduit à un mélange d'acide 4,4,4-trifluoro-3(S)-hydroxybutanoïque et de 4,4,4-trifluoro-3(R)-hydroxybutanoate d'éthyle, et extraction de l'énantiomère (R) non hydrolysé.

Le procédé de l'invention est représenté dans le schéma 1 suivant :

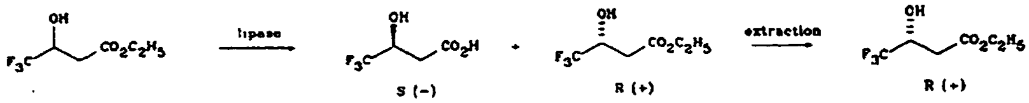

S (-) R (+) R (+)

schéma 1

Selon une forme particulière de l'invention la lipase peut être immobilisée sur résine.

La lipase utilisée est par exemple la lipase de Candida cylindracea (lipase-MY, Meito Sangyo Co. Ltd., Japon),

la lipase de Candida (lipase-AY, Amano, Japon) ou, de préférence, la lipase de Candida antarctica, en particulier immobilisée sur résine (Novozym® 435, Novo Nordisk, Danemark).

L'hydrolyse est réalisée en milieu aqueux, par exemple dans une solution tampon phosphate ou dans une solution aqueuse de bicarbonate de potassium, à une température comprise entre 20 et 40°C, pendant 1 à 18 heures.

On utilise le Novozym® 435 à une concentration de 0,1 à 10 % et les lipases AY et MY à des concentrations de 5 à 20% en poids par rapport au poids de substrat.

L'énantiomère (R) non hydrolysé est récupéré par extraction dans un solvant organique, alors que l'énantiomère (S), hydrolysé en acide, reste dans la phase aqueuse à pH basique.

Le 4,4,4-trifluoro-1,3(R)-butanediol est ensuite obtenu en une seule étape par réduction du 4,4,4-trifluoro-3(R)-hydroxybutanoate d'éthyle.

Les exemples 1 et 2 illustrent l'invention.

Exemple 1 : 4,4,4-trifluoro-3(R)-hydroxybutanoate d'éthyle

A 2 g de (+)-4,4,4-trifluoro-3-hydroxybutanoate d'éthyle en suspension dans 50 ml de tampon phosphate 0,01 M ($KH_2PO_4$ + $Na_2HPO_4$), pH = 7,2, on ajoute 3 mg de Novozym® 435, puis on agite le mélange réactionnel pendant 17 h, à 22°C, en maintenant le pH constant par addition d'une solution aqueuse d'hydroxyde de sodium 1M.
On extrait ensuite à pH = 9 par du dichlorométhane, puis on réunit les phases organiques, qui sont ensuite séchées sur sulfate de magnésium, filtrées et évaporées sous vide.
On obtient ainsi 0,74 g de produit (Rendement = 74 %).
$[\alpha]_D^{20}$ = + 15° (chloroforme, c = 0,914)
ee : > 98% (HPLC chiralcel OD-R).

Exemple 2 : 4,4,4-trifluoro-3(R)-hydroxybutanoate d'éthyle

On introduit dans un réacteur de 10 litres, 600 g de (±)-4,4,4-trifluoro-3-hydroxybutanoate d'éthyle, 6 litres d'eau déminéralisée, 323 g de bicarbonate de potassium et 30 g de Novozym® 435. On agite le mélange pendant 1 h 10 mn, puis on filtre l'enzyme sur verre fritté et on ajoute 1 kg de chlorure de sodium au filtrat. On agite le mélange jusqu'à dissolution puis on extrait le milieu avec 2 fois 2 litres puis 2 fois 1 litre de dichlorométhane. On lave l'ensemble des phases organiques avec 1 litre d'eau saturée par du chlorure de sodium puis on évapore le solvant à pression atmosphérique. On obtient 205,4 g de produit contenant 12 % de dichlorométhane (Rendement corrigé : 60,2 %).
$[\alpha]_D^{20}$ = + 14,59° (chloroforme, c = 1)
ee = 96,7 % (HPLC Chiralpack AD)

Le 4,4,4-trifluoro-3(R)-hydroxybenzoate d'éthyle peut ensuite être transformé en 4,4,4-trifluoro-1,3(R)-butanediol, en une seule étape.

L'exemple 3 illustre l'utilisation du 4,4,4-trifluoro-3(R)-hydroxybenzoate d'éthyle pour la préparation du 4,4,4-trifluoro-1,3(R)-butanediol.

Exemple 3 : 4,4,4-trifluoro-1,3(R)-butanediol

Sur une suspension de 20,4 g de borohydrure de sodium dans 270 ml d'éthanol absolu, on verse goutte à goutte, sous argon, en ne dépassant pas 35°C, une solution de 100 g de 4,4,4-trifluoro-3(R)-hydroxybutanoate d'éthyle dans 80 ml d'éthanol absolu. On agite ensuite le mélange réactionnel pendant 16 h puis on le verse dans de l'eau glacée et on ajoute 150 ml d'une solution aqueuse d'acide chlorhydrique concentré. On extrait ensuite avec 3 fois 250 ml d'acétate d'éthyle, on sèche la phase organique sur sulfate de sodium, on la filtre et on l'évapore sous vide. On obtient 64,6 g d'un liquide trouble qui est purifié par distillation sous vide. On obtient finalement 53 g de produit.
Rendement : 69 %.
Point d'ébullition : 68-75°C (sous 66 Pa, soit 0,5 mm Hg).
$[\alpha]_D^{20}$ = + 26,1° (c = 0,548 ; chloroforme).

Cette nouvelle méthode, qui permet d'obtenir le 4,4,4-trifluoro-1,3(R)-butanediol en trois étapes seulement, dont une simple extraction, à partir du (±)-4,4,4-trifluoro-3-hydroxybutanoate d'éthyle et avec un rendement élevé, améliore la synthèse de la béfloxatone.

## Revendications

1. Procédé de préparation du 4,4,4-trifluoro-3(*R*)-hydroxy-butanoate d'éthyle **caractérisé en ce qu'**il comprend les étapes de

(a) hydrolyse sélective de la fonction ester de l'énantiomère (S) compris dans le mélange racémique (±) -4,4,4-trifluoro-3-hydroxybutanoate d'éthyle au moyen d'une lipase et
(b) extraction de l'énantiomère *(R)* non hydrolysé.

**2.** Procédé selon la revendication 1, **caractérisé en ce que** la lipase est immobilisée sur résine.

**3.** Procédé selon la revendication 2, **caractérisé en ce que** la lipase est une lipase de Candida antarctica.

**4.** Procédé selon la revendication 3, **caractérisé en ce que** la lipase est utilisée à une concentration de 0,1 à 10 % en poids par rapport au poids de substrat.

**5.** Procédé selon la revendication 1, **caractérisé en ce que** la lipase est une lipase de Candida cylindracea.

**6.** Procédé selon la revendication 5, **caractérisé en ce que** la lipase est utilisée à une concentration de 5 à 20 % en poids par rapport au poids de substrat.

**7.** Procédé de préparation du 4,4,4-trifluoro-3(*R*)-hydroxybutanoate d'éthyle selon l'une quelconque des revendications 1 à 6, **caractérisé en ce qu'**il est suivi de l'étape supplémentaire de synthèse qui consiste en la réduction du 4,4,4-trifluoro-3(*R*)-hydroxybutanoate d'éthyle pour donner le 4,4,4-trifluoro-1,3(*R*)-butanediol.

## Patentansprüche

**1.** Verfahren zur Herstellung von Ethyl-4,4,4-trifluoro-3(*R*)-hydroxybutanoat, **dadurch gekennzeichnet, daß** es die Schritte umfaßt

(a) selektive Hydrolyse der Esterfunktion des (S)-Enantiomers, welches in der racemischen Mischung von Ethyl-(±)-4,4,4-trifluoro-3-hydroxybutanoat enthalten ist, mittels einer Lipase und
(b) Extraktion des nicht-hydrolysierten (*R*)-Enantiomers.

**2.** Verfahren nach Anspruch 1, **dadurch gekennzeichnet, daß** die Lipase auf einem Harz immobilisiert ist.

**3.** Verfahren nach Anspruch 2, **dadurch gekennzeichnet, daß** die Lipase eine Lipase aus Candida antarctika ist.

**4.** Verfahren nach Anspruch 3, **dadurch gekennzeichnet, daß** die Lipase in einer Konzentration von 0,1 bis 10 Gew.-% bezogen auf das Gewicht des Substrats verwendet wird.

**5.** Verfahren nach Anspruch 1, **dadurch gekennzeichnet, daß** die Lipase eine Lipase aus Candida cylindracea ist.

**6.** Verfahren nach Anspruch 5, **dadurch gekennzeichnet, daß** die Lipase in einer Konzentration von 5 bis 20 Gew.-% bezogen auf das Gewicht des Substrats verwendet wird.

**7.** Verfahren zur Herstellung von Ethyl-4,4,4-trifluoro-3(*R*)-hydroxybutanoat nach mindestens einem der Ansprüche 1 bis 6, **dadurch gekennzeichnet, daß** ein zusätzlicher Syntheseschritt folgt, der in der Reduktion von Ethyl-4,4,4-trifluoro-3(*R*)-hydroxybutanoat unter Bildung von 4,4,4-Trifluoro-1,3(*R*)-butandiol besteht.

## Claims

**1.** Process for the preparation of ethyl 4,4,4-trifluoro-3(R)-hydroxybutanoate, **characterized in that** it comprises the stages of

(a) selective hydrolysis by means of a lipase of the ester functional group of the (S) enantiomer included in the racemic mixture ethyl (±)-4,4,4-trifluoro-3-hydroxybutanoate and
(b) extraction of the unhydrolysed (R) enantiomer.

**2.** Process according to Claim 1, **characterized in that** the lipase is immobilized on resin.

3. Process according to Claim 2, **characterized in that** the lipase is a Candida antarctica lipase.

4. Process according to Claim 3, **characterized in that** the lipase is used at a concentration of 0.1 to 10% by weight with respect to the weight of substrate.

5. Process according to Claim 1, **characterized in that** the lipase is Candida cylindracea lipase.

6. Process according to Claim 5, **characterized in that** the lipase is used at a concentration of 5 to 20% by weight with respect to the weight of substrate.

7. Process for the preparation of ethyl 4,4,4-trifluoro-3(R)-hydroxybutanoate according to any one of Claims 1 to 6, **characterized in that** it is followed by the additional synthetic stage which consists of the reduction of the ethyl 4,4,4-trifluoro-3(R)-hydroxybutanoate to give 4,4,4,-trifluoro-1,3(R)-butanediol.